# EUROPEAN PATENT SPECIFICATION

(11) **EP 4 551 221 B1**
(45) Date of publication and mention of the grant of the patent: **18.02.2026**
(21) Application number: 23735068.1
(22) Date of filing: 05.07.2023
(51) Int. Cl.: A61K 31/46, A61P 15/00, A61P 15/02, A61P 15/10

(54) **COMPOUND FOR TREATMENT OF FEMALE SEXUAL DYSFUNCTION**
VERBINDUNG ZUR BEHANDLUNG DER WEIBLICHEN SEXUELLEN DYSFUNKTION
COMPOSÉ DESTINÉ AU TRAITEMENT DES DYSFONCTIONNEMENTS SEXUELS CHEZ LA FEMME

(30) Priority: 08.07.2022 EP 22183862
(43) Date of publication of application: 14.05.2025
(73) Proprietor: Initiator Pharma A/S, 2200 Copenhagen N (DK)
(72) Inventor: SIMONSEN, Ulf, 2200 Copenhagen N (DK); COMERMA-STEFFENSEN, Simon, 2200 Copenhagen N (DK)
(74) Representative: Høiberg P/S
(86) International application number: PCT/EP2023/068583
(87) International publication number: WO 2024/008808

(56) References cited:
- WO-A1-2011/092061
- HIGGINS AGNES ET AL: "Antidepressant-associated sexual dysfunction: impact, effects, and treatment", DRUG, HEALTHCARE AND PATIENT SAFETY, vol. 2, 1 January 2010 (2010-01-01), pages 141 - 150, XP055858641, Retrieved from the Internet <URL:https://www.ncbi.nlm.nih.gov/pmc/articles/PMC3108697/pdf/dhps-2-141.pdf> DOI: 10.2147/DHPS.S7634

## Description

### Technical field

The present disclosure relates to treatment of female sexual dysfunction using a small-molecule drug.

### Background

Female sexual dysfunction (FSD) is a prevalent problem, afflicting approximately 40% of women and there are few treatment options (Allahdadi et al. 2009). FSD is more typical as women age and is a progressive and widespread condition. Common symptoms associated with FSD include diminished vaginal lubrication, pain and discomfort upon intercourse, decreased sense of arousal and difficulty in achieving orgasm. Only a small percentage of women seek medical attention. In comparison to the overwhelming research and treatment for erectile dysfunction in males, specifically with the development of phosphodiesterase type 5 inhibitors, significantly less has been explored regarding FSD. The treatment is primarily limited to psychological therapy, although different pharmacological modalities have been developed though with low efficacy and adverse effects (Miller et al., 2018).

WO 2011/092061 discloses monoamine reuptake inhibitors and proposes them for treatment of for treatment of CNS conditions. The compound of formula (I) is exemplified as a monoamine reuptake inhibitor.

Higgins et al. (Drug, Healthcare and Patient Safety 2010:2 141-150) discloses that sexual dysfunction is a common side effects of antidepressants, particularly of selective serotonin reuptake inhibitor (SSRIs) and serotonin norepinephrine reuptake inhibitor (SNRls).

### Summary

The invention is defined by the claims. Any subject matter falling outside the scope of the claims is provided for information purposes only.

As outlined above, a compound able to treat FSD is highly desired. The present disclosure provides for compounds and compositions useful in the treatment of female sexual dysfunction.

The present inventors have surprisingly found that the compound of formula (I) as described herein is able to improve parameters associated with improved sexual function and sexual behaviour related to FSD in female animal models. The examples herein unexpectedly demonstrate that the compound of formula (I) induces an increase in blood flow to the clitoris similar to the effect of clitoral nerve stimulation, as well as inducing an increase in genital temperature by raising clitoral temperature. In addition, the inventors have shown that the compound of formula (I) induces increase in para-copulatory behaviour, or "courting", in female rats.

Thus, the present invention demonstrates that the compound of formula (I) addresses both physical and behavioural components of FSD, which can translate into effects in sexual interest, arousal, sexual desire, and sexual pain associated with FSD.

Thus, in one main aspect, a compound of formula (I) is provided: 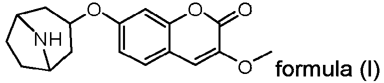 or a pharmaceutically acceptable salt thereof, for use in the treatment, prevention, or alleviation of female sexual dysfunction in a subject.

One aspect provides for a pharmaceutical composition comprising a compound of formula (I): 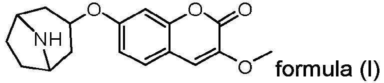 or a pharmaceutically acceptable salt thereof, for use in the treatment, prevention, or alleviation of female sexual dysfunction in a subject.

The references to methods of treatment in this description are to be interpreted as references to the compounds, pharmaceutical compositions and medicaments of the present invention for use in a method for the treatment of the human (or animal) body by therapy.

Additionally disclosed is a use of a compound of formula (I), 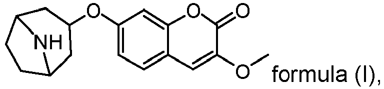 for the manufacture of a medicament for the treatment of female sexual dysfunction.

In another aspect, a method is provided for the treatment, prevention, or alleviation of female sexual dysfunction, said method comprising administering to a subject in need thereof a therapeutically effective amount of the compound of formula (I) or a pharmaceutically acceptable salt thereof.

In yet another aspect, a compound of formula (I), or a pharmaceutically acceptable salt thereof, for use as described herein is provided to increase the genital temperature in a subject in need thereof by administering a therapeutically effective amount of the compound of formula (I) or a pharmaceutically acceptable salt thereof.

In another aspect, a compound of formula (I), or a pharmaceutically acceptable salt thereof, for use as described herein is provided to increase genital blood flow in a subject in need thereof by administering a therapeutically effective amount of a compound of formula (I) or a pharmaceutically acceptable salt thereof.

In another aspect, a compound of formula (I), or a pharmaceutically acceptable salt thereof, for use as described herein, is provided for increasing sexual desire, sexual interest, sexual arousal, lubrication, sexual satisfaction, or a combination thereof in a subject by administering a therapeutically effective amount of a compound of formula (I), or a pharmaceutically acceptable salt thereof.

In another aspect, a compound of formula (I), or a pharmaceutically acceptable salt thereof, for use as described herein is provided for reducing difficulty in reaching orgasm upon sexual stimulation or intercourse in a subject by administering a therapeutically effective amount of a compound of formula (I), or a pharmaceutically acceptable salt thereof.

In a final aspect, a compound of formula (I), or a pharmaceutically acceptable salt thereof, for use as described herein is provided for reducing sexual pain in a subject by administering a therapeutically effective amount of a compound of formula (I), or a pharmaceutically acceptable salt thereof.

### Description of Drawings

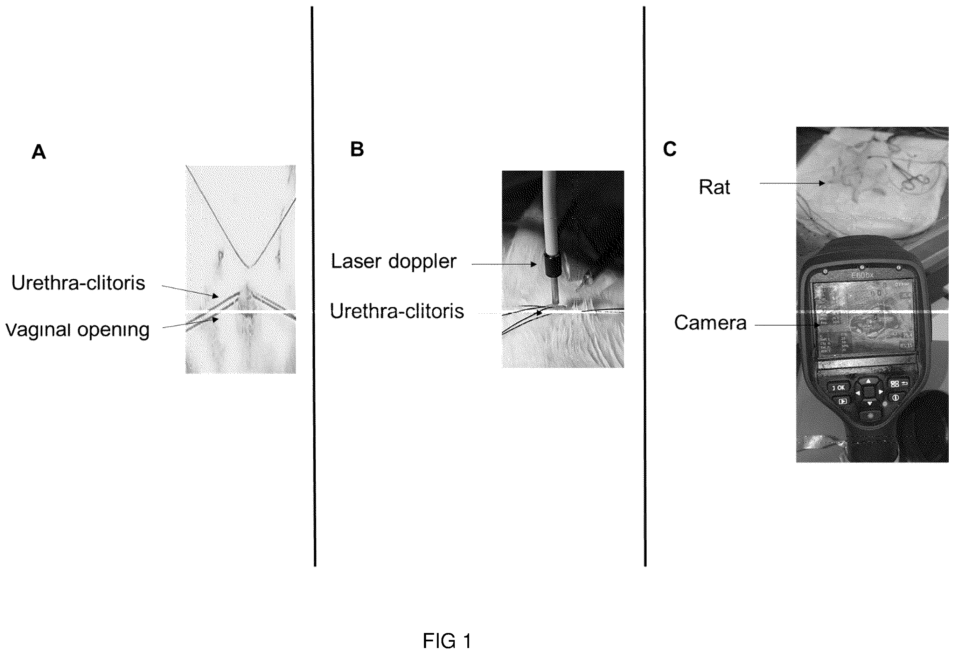

Figure 1: Preparation of the female rat. **A.** Female genital area of interest. **B.** Positioning of laser doppler. **C.** Positioning of the thermographic camera.

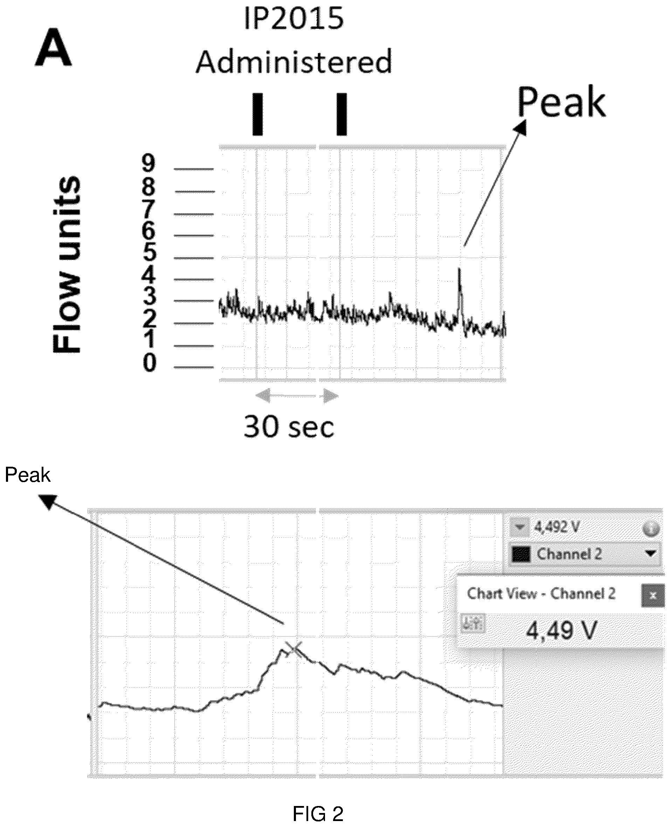

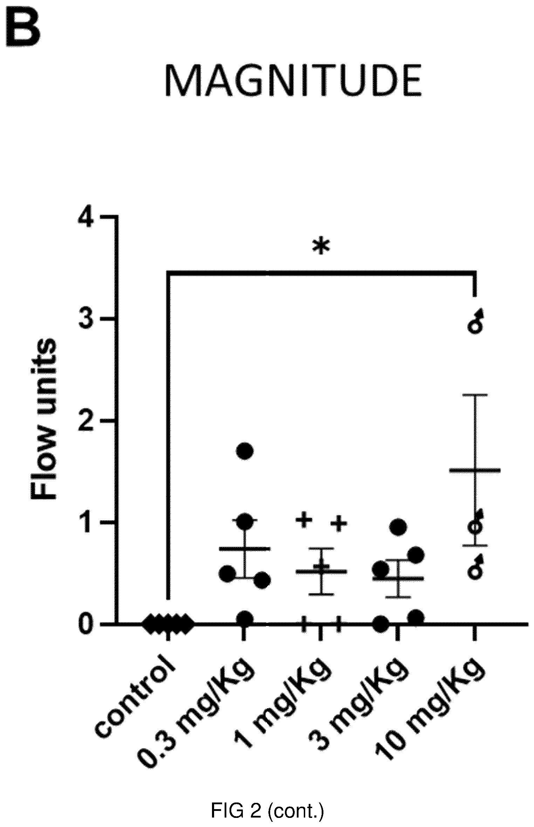

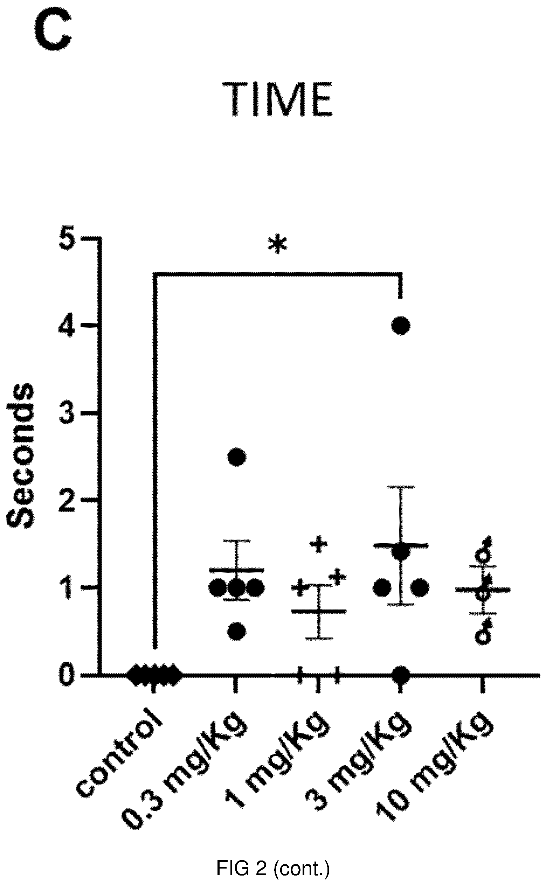

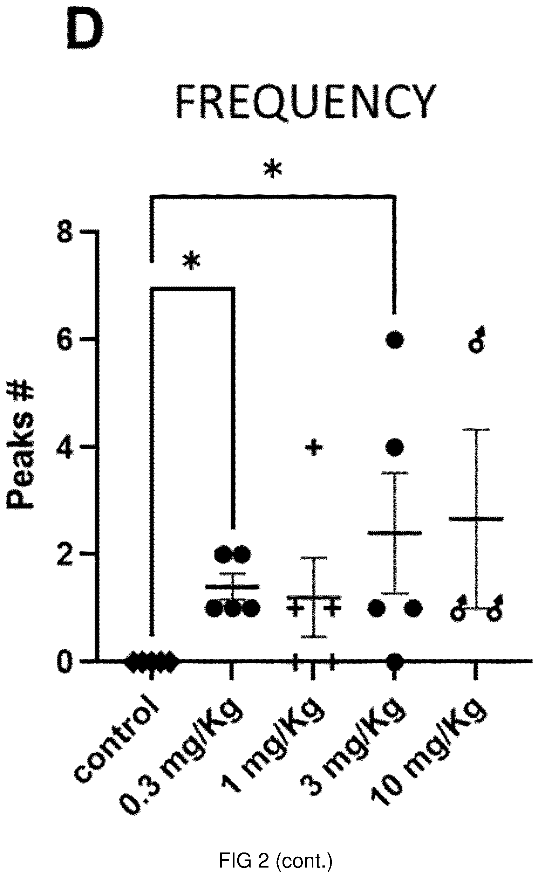

Figure 2: Laser Doppler experimental data and averages of IP2015 infusion doses at the female genital organs. **A.** Experimental visualization of the IP2015 effect after administration. **B.** IP2015 average magnitude response changes due to IP2015 different administered doses. **C.** IP2015 average time response changes due to IP2015 different administered doses. **D.** IP2015 average frequency response changes due to IP2015 different administered doses.

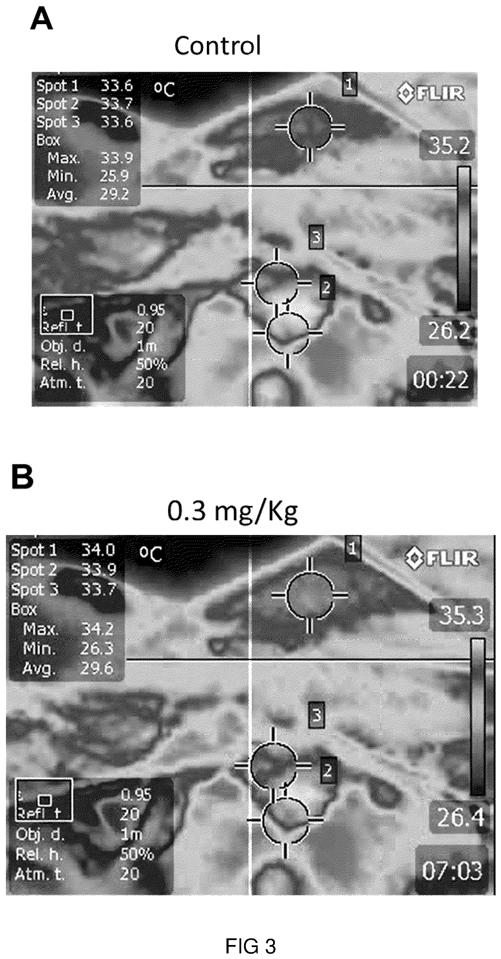

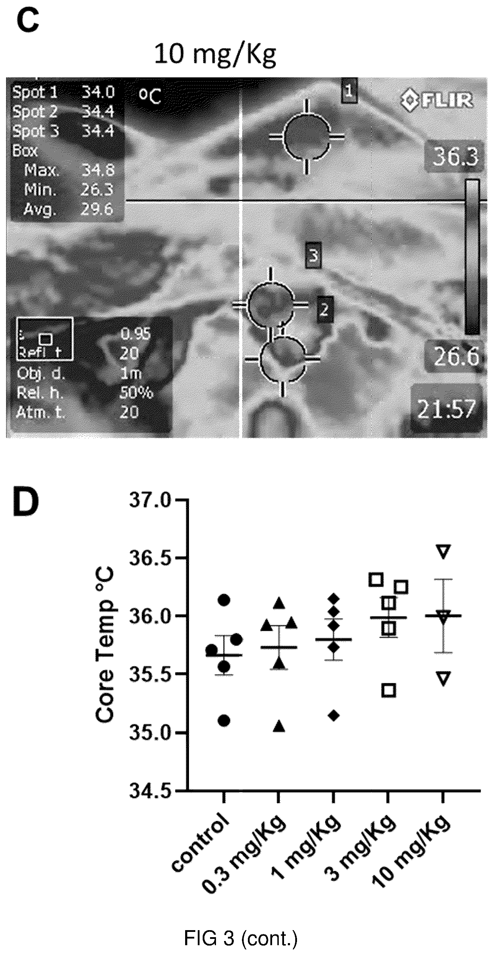

Figure 3: Thermographic images at 4x magnification of IP2015 infusion in the rat female genital organ changes. **A.** Thermographic view of the control/basal. **B.** Thermographic view of the infusion of 0.3 mg/Kg of IP2015. **C.** Thermographic view of the infusion of 10 mg/Kg of IP2015. **D.** Anal/core temperature taken under IP2015 infusion of doses.

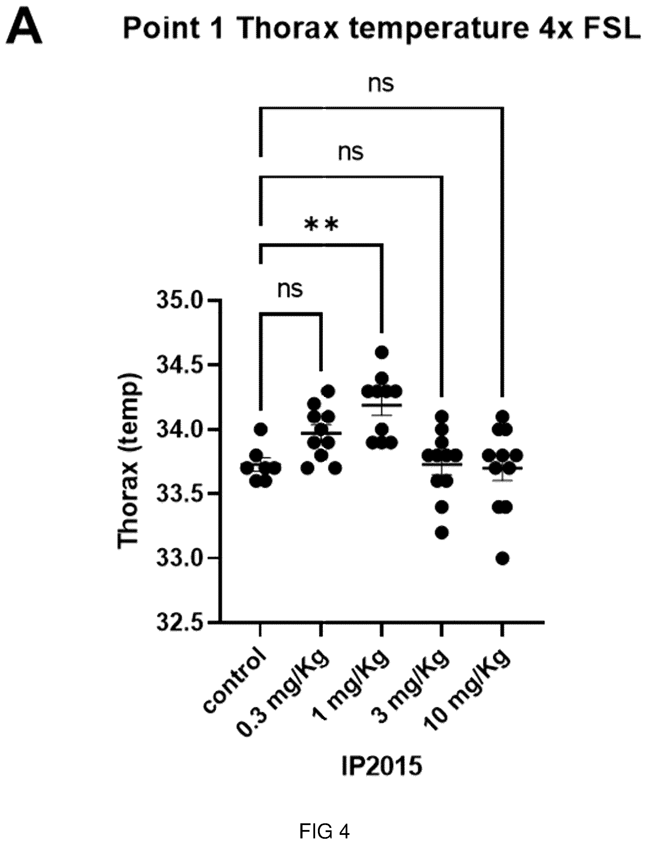

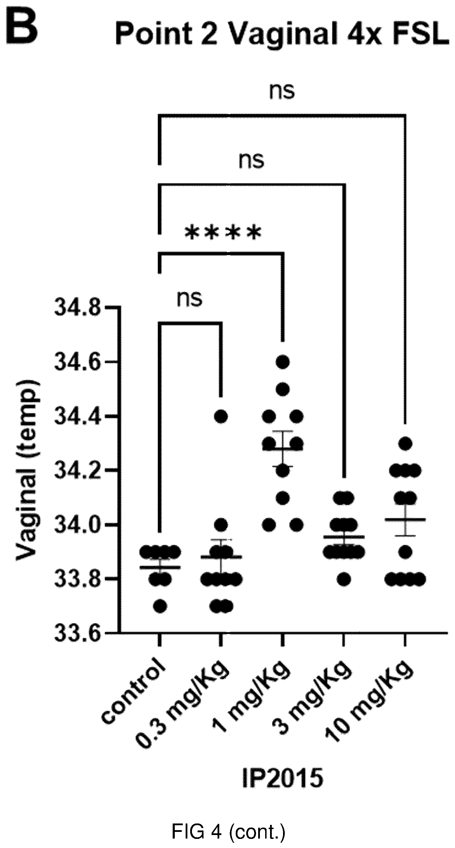

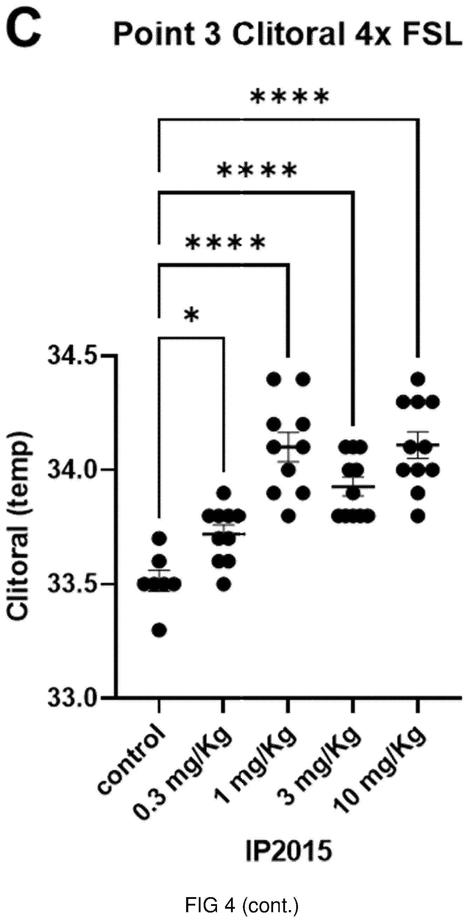

Figure 4: Thermographic averages of IP2015 infusion in the rat female genital organ and related structures. **A.** Quantified Chest/Thoracic thermographic values obtained during the infusion of IP2015 doses. **B.** Quantified vaginal thermographic values obtained during the infusion of IP2015 doses. **C.** Quantified Clitoral/Urethra thermographic values obtained during the infusion of IP2015 doses. One Way ANOVA comparisons by Tukey Post hoc test. P<0.05 *.

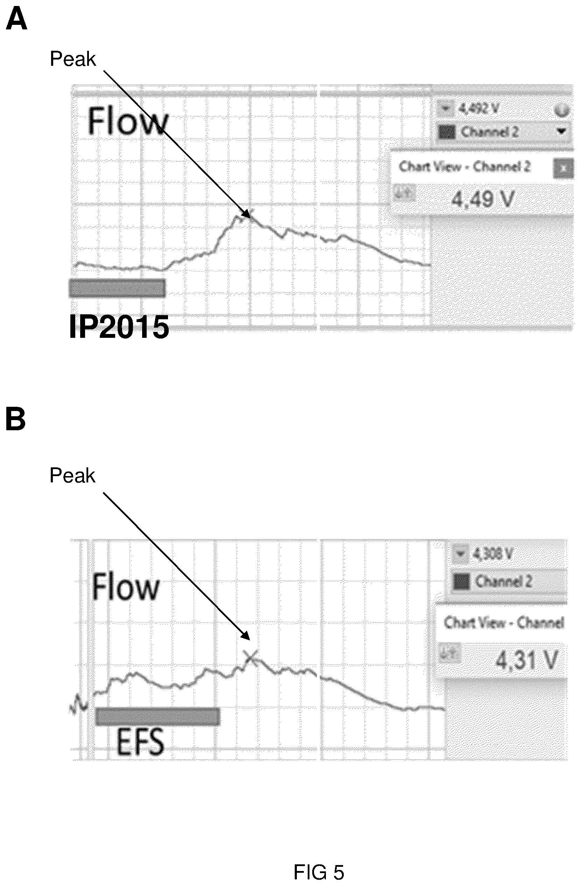

Figure 5. **A.** Representative original laser Doppler trace showing IP2015 infusion increases clitoral blood flow in an anaesthetized female rat. A bolus IP2015 (1 mg/kg) was infused over 1 min in the jugular vein (shown as grey bar). IP2015 increases blood flow measured with laser Doppler in the clitoral cavernous tissue. The duration of the recording is 5 min. **B.** Representative original laser Doppler trace showing stimulation of the cavernous nerve to clitoris increases blood flow in anaesthetized female rat to comparable levels as IP2015 infusion doses. The cavernous nerve was stimulated with electrical field stimulation (EFS, 10 Hz, 1 msec pulse, 6V, shown as grey bar). EFS increases flow measured with laser Doppler in the cavernous tissue. The duration of the recording is 5 min.

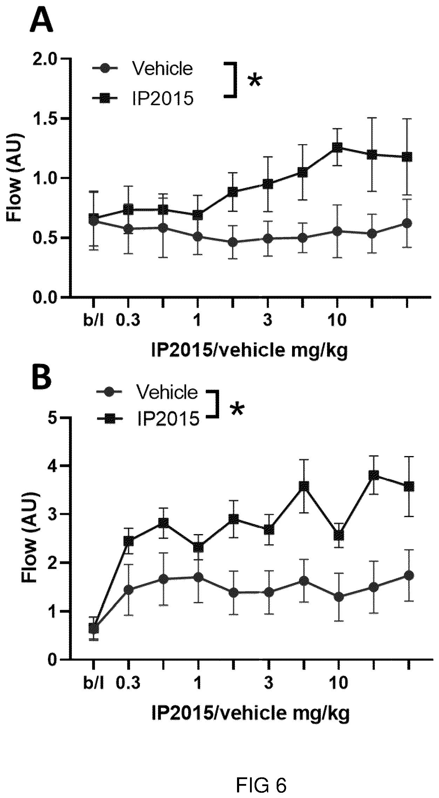

Figure 6. Average effect of increasing doses of IP2015 on clitoral blood flow in female rats. **A.** Average mean clitoral blood flow, and **B.** peak clitoral blood flow. IP2015 dose-dependently increases both average and peak blood flow in the clitoral area. Data are presented as means±SEM, n=6 and n=5 rats infused, with IP2015 and vehicle respectively. Statistical significance defined as *p<0.05, versus vehicle, compared via 2-way ANOVA.

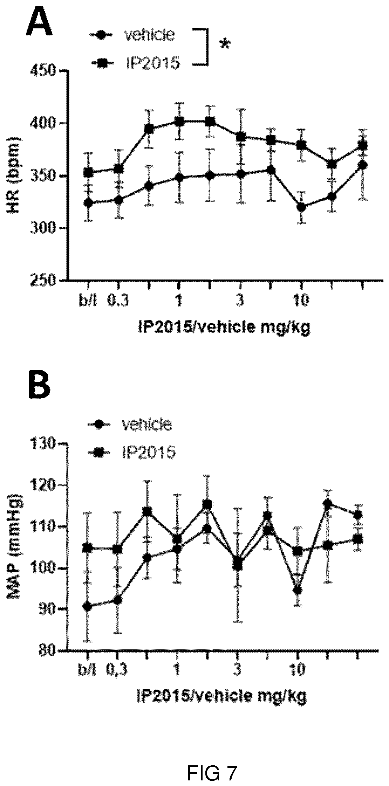

Figure 7. Average effect of increasing doses of IP2015 on heart rate and blood pressure in female rats. **A.** Heart rate and **B.** Mean arterial blood pressure (MAP) at baseline (b/l) and after infusion of increasing doses of vehicle and IP2015. Data are presented as means±SEM, n=6 and n=5 rats infused, with IP2015 and vehicle respectively. Statistical significance defined as **p*<0.05, versus vehicle, compared via 2-way ANOVA.

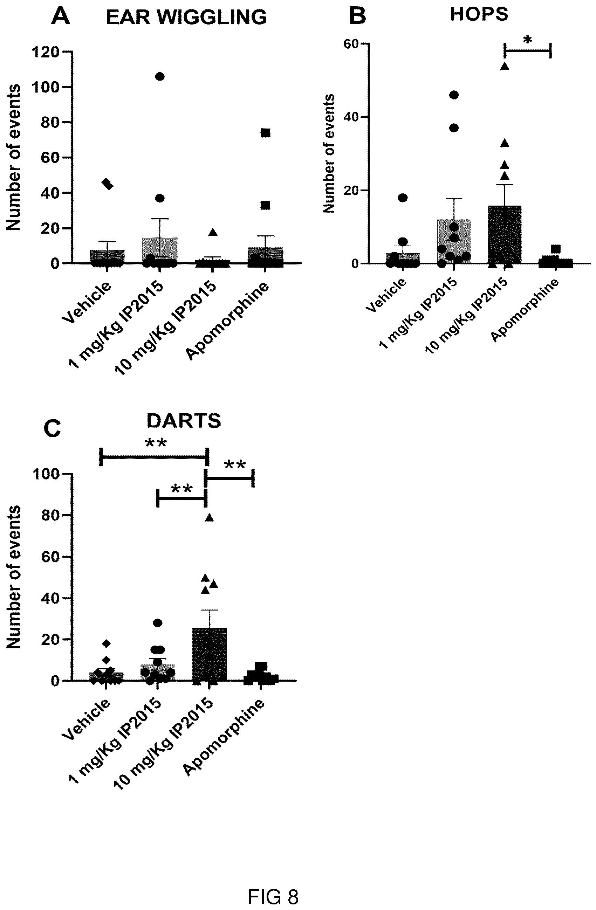

Figure 8. IP2015 increases sexual behaviour in female rats with synchronized sexual in estrus. Effect of single doses of vehicle, IP2015 (1 and 10 mg/kg), and apomorphine on sexual behavioural parameters in female rats. **(A)** Effect of IP2105 on ear wiggling, **(B)** effect of IP2015 on number of hops, and **(C)** effect of IP2015 on darts in synchronized female rats. Data are presented as means±s.e.mean of n=10-12 rats. The one-way ANOVA was significant for hops, and post-test showed the number of hops were significant in IP2015 (10 mg/kg) compared to apomorphine treatment. **P<0.01 for number of darts in rats treated with IP2015 (10 mg/kg) versus vehicle and apomorphine.

### Definitions

As used herein, the terms "female sexual dysfunction" or "FSD" refer generally to the impairment of the sexual function in a female. Sexual dysfunction in females includes inhibited orgasm. Female sexual dysfunction includes, but is not limited to, a number of categories of diseases, conditions and disorders including female hypoactive sexual desire disorder/dysfunction (FHSDD or HSDD, used interchangeably herein), female orgasmic disorder/dysfunction (FOD), sexual anhedonia, female sexual interest/arousal disorder/dysfunction (FSIAD), female sexual arousal disorder/dysfunction (FSAD), sexual pain disorder/dysfunction and dyspareunia.

As used herein, the terms "female sexual function index" or "FSFI" refer generally to the 19-item patient reported outcome (PRO) instrument/questionnaire that has been used in clinical trials to measure overall sexual function (Rosen R, Brown C, et al., 2000) and grade the severity of FSD,wherein, individual domain questions from the FSFI have been used to measure specific components of sexual function, including:
A) Sexual desire domain (FSFI-D): (FSFI-; questions 1-2 (frequency, level)
B) Sexual arousal domain: questions 3-6 (level, confidence, satisfaction)
C) Lubrication domain: questions 7-10 (frequency, difficulty, frequency of maintaining, difficulty in maintaining)
D) Orgasm domain: questions 11-13 (frequency, difficulty, satisfaction)
E) Satisfaction domain: questions 14-16 (with amount of closeness with partner, with sexual relationship, with overall sex life)
F) Pain domain: questions 17-19 (frequency during vaginal penetration, frequency following vaginal penetration, level during or following vaginal penetration).

Responses are evaluated in a 5 point scale (1 to 5), where higher numbers indicate a higher level of said specific components of sexual function and lower numbers indicate a lower level of said specific components of sexual function. Thus, lower numbers indicate more severe levels of sexual dysfunction. Full-scale FSFI and/or domain-level scoring and interpretation can be utilised to measure overall sexual function and grade the severity of FSD.

As used herein, the terms " Female Sexual Distress Scale-Desire/Arousal/Orgasm " or "FSDS-DAO" refer generally to the 15-item patient reported outcome (PRO) instrument/questionnaire that has be used in clinical trials to measure overall sexual function and grade the severity of female hypoactive sexual desire dysfunction (FHSDD) (Derogatis et al. 2008, Derogatis et al. 2021). The total score may be calculated as the sum of the responses and ranges from 0 to 60, with higher scores indicating a greater level of distress. The FSDS-DAO was adapted from the validated, revised female sexual distress scale (FSDS-R) questionnaire. Question 13 of the FSDS-DAO (FSDS-DAO Q13) asks patients, "How often did you feel: Bothered by low sexual desire?". Responses can be graded on a scale of 0 (never) to 4 (always). A decrease in the FSDS-DAO Q13 score over time denotes improvement in the level of distress associated with low sexual desire. Question 13 (Q13) of the FSDS-DAO questionnaire is identical to question 13 of the FSDS-R questionnaire.

### Detailed description

### Compounds and compositions

One embodiment of the present disclosure provides for a compound of formula (I), 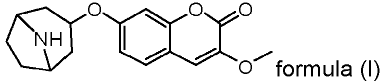 or a pharmaceutically acceptable salt thereof, for use in the treatment, prevention, or alleviation of female sexual dysfunction in a subject. The compound of formula (I) is a monoamine reuptake inhibitor. Compounds may be tested for their ability to inhibit reuptake of the monoamines dopamine, noradrenaline and serotonin in synaptosomes e.g. such as described in WO 97/30997 or WO 97/16451. The compound of formula (I) hydrochloride has been reported with the IC₅₀ values: 0.0029 µM (serotonin) 0.07 µM (dopamine) 0.0038 µM (noradrenaline) (US 9,133,184 B1).

In one embodiment, the compound of formula (I) has the structure of formula (Ia); 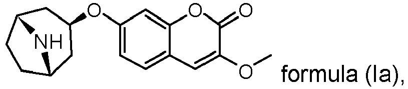 or a pharmaceutically acceptable salt thereof.

In one embodiment, the compound is 7-[(8-azabicyclo[3.2.1]octan-3-yl)oxy]-3-methoxy-chromen-2-one, or a pharmaceutically acceptable salt thereof. In one embodiment, the compound is exo-7-[(8-azabicyclo[3.2.1]octan-3-yl)oxy]-3-methoxy-chromen-2-one, or a pharmaceutically acceptable salt thereof. In one embodiment, the name "IP2015" means the compound of formula I. In one embodiment, the name "IP2015" means the compound of formula la. In a specific embodiment of the present disclosure "IP2015" means the hydrochloride of the compound of formula I.

In one embodiment, the pharmaceutically acceptable salt is of an organic or an inorganic counterion. In one embodiment, the pharmaceutically acceptable salt is selected from the list consisting of the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate, the phthalate, the salicylate, the sorbate, the stearate, the succinate, the tartrate, the toluene-p-sulphonate. Other pharmaceutically acceptable salts are known to those of skill in the art. Such salts may be formed by procedures well known and described in the art.

In one embodiment of the disclosure, the compound is exo-7-[(8-azabicyclo[3.2.1]octan-3-yl)oxy]-3-methoxy-chromen-2-one hydrochloride.

The compound of the present disclosure is a monoamine reuptake inhibitor. Compounds may be tested for their ability to inhibit reuptake of the monoamines dopamine, noradrenaline and serotonin in synaptosomes e.g. such as described in WO 97/30997 or WO 97/16451. The compound of formula (I) hydrochloride has been reported with the IC₅₀ values: 0.0029 µM (serotonin) 0.07 µM (dopamine) 0.0038 µM (noradrenaline) (US 9,133,184 B1).

One embodiment of the present disclosure provides for a pharmaceutical composition, comprising a therapeutically effective amount of a compound of formula (I), 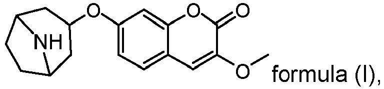 and at least one pharmaceutically acceptable carrier, excipient or diluent for use in the treatment, prevention, or alleviation of female sexual dysfunction in a subject. In the context of the present disclosure, any mention of administration or use of a compound of the disclosure is to be construed as also extending to administration or use of a composition of disclosure.

In one embodiment, the composition for use as described herein is a pharmaceutical composition. In one embodiment of the present disclosure, the composition for use as described herein is formulated for oral, rectal, bronchial, nasal, pulmonal, topical (including buccal and sub-lingual), transdermal, vaginal or parenteral (including cutaneous, mucosal, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems.

### Female sexual dysfunction

The compounds of the present disclosure have shown potential in the treatment of female sexual dysfunction (FSD).

Thus, one embodiment of the present disclosure provides for a compound of formula (I), or a pharmaceutically acceptable salt thereof, for use in the treatment of female sexual dysfunction.

Female sexual dysfunction can for example be of physiological or psychological origin.

In one embodiment the female sexual dysfunction is selected from the group consisting of:
(i) female sexual interest/arousal disorder (FSIAD),
(ii) hypoactive sexual desire dysfunction,
(iii) female sexual arousal dysfunction
(iv) orgasmic dysfunction,
(v) sexual pain disorder, and
(vi) dyspareunia.

As shown in the examples, administration the compound of formula (I) increases clitoral blood flow similarly to clitoral nerve stimulation. In addition, the compound of formula (I) is able to increase clitoral temperature or vaginal temperature. These effects are also marks of normal sexual arousal, where increased blood flow to the clitoral cavernosal and labial arteries result in increased clitoral intracavernous pressure, tumescence, protrusion of the glans clitoris, and eversion and engorgement of the labia minora (Bergman., 2005). Therefore, increased genital blood flow and temperature as shown in the examples is linked to increased genital lubrication, increased sexual arousal, desire satisfaction and orgasm.

Subjects suffering from female sexual arousal disorder, (female sexual interest disorder; sexual arousal disorder) presents with little or no interest in sex and do not respond subjectively or physically to sexual stimulation. The decrease in interest and ability to be sexually aroused is greater than what might be expected based on a subject's age and the relationship duration. Lack of sexual interest and inability to be sexually aroused are considered a disorder if they distress the subject and if interest is absent throughout the sexual experience. In one embodiment, FSD is as defined in the Diagnostic and Statistical Manual of Mental Disorders (DSM), 4th edition. In one embodiment of the disclosure, the FSD is female sexual interest/arousal disorder (FSIAD), which encompasses FSAD and FHSDD. FOD and FSIAD are defined in the Diagnostic and Statistical Manual of Mental Disorders (DSM), 5th edition.

The diagnostic criteria for FSIAD include a lack of, or significantly reduced, sexual interest/arousal, as manifested by at least three of the following: (1) absent/reduced interest in sexual activity; (2) absent/reduced sexual/erotic thoughts or fantasies; (3) no/reduced initiation of sexual activity, and typically unreceptive to a partner's attempts to initiate; (4) absent/reduced sexual excitement/pleasure during sexual activity in almost all or all sexual encounters; (5) absent/reduced sexual interest/arousal in response to any internal or external sexual/erotic cues; and (6) absent/reduced genital or nongenital sensations during sexual activity in almost all or all sexual encounters. In FSIAD, these symptoms have persisted for a minimum duration of approximately six months and cause the subject significant distress.

As shown in the examples, the compound of formula (I) produces an increase in para-copulatory or courting behaviours in female rats as seen by an increase in hops and darts in female rats. Thus, highlighting the compound's ability to increase sexual interest or desire to engage in sexual activity.

Female hypoactive sexual desire dysfunction (FHSDD) is characterised by absence or marked reduction in desire or motivation to engage in sexual activity as manifested by any of the following: 1) reduced or absent spontaneous desire (sexual thoughts or fantasies); 2) reduced or absent responsive desire to erotic cues and stimulation; or 3) inability to sustain desire or interest in sexual activity once initiated. The pattern of diminished or absent spontaneous or responsive desire or inability to sustain desire or interest in sexual activity has occurred episodically or persistently over a period at least several months, and is associated with clinically significant distress. In one embodiment of the present disclosure, the FSD is hypoactive sexual desire dysfunction.

Hypoactive sexual desire disorder/dysfunction includes a disorder in which sexual fantasies and desire for sexual activity are persistently or recurrently diminished or absent, causing marked distress or interpersonal difficulties. Sexual anhedonia includes decreased or absent pleasure in sexual activity. Sexual arousal disorder/dysfunction can be caused by reduced oestrogen, illness, or treatment with diuretics, antihistamines, antidepressants, or antihypertensive agents.

In one embodiment, the hypoactive sexual desire dysfunction is lifelong hypoactive sexual desire dysfunction or acquired hypoactive sexual desire dysfunction. In one embodiment of the disclosure, the hypoactive sexual desire dysfunction is generalised hypoactive sexual desire dysfunction or situational hypoactive sexual desire dysfunction. Lifelong, generalised hypoactive sexual desire dysfunction is characterised by the subject always having experienced hypoactive sexual desire dysfunction from the time of initiation of relevant sexual activity and the desired response is currently absent or diminished in all circumstances, including masturbation. Lifelong, situational hypoactive sexual desire dysfunction is characterised by the subject always having experienced hypoactive sexual desire dysfunction, from the time of initiation of relevant sexual activity and the desired response is currently absent or diminished in some circumstances, with some partners, or in response to some stimuli, but not in other situations. Generalised, acquired hypoactive sexual desire dysfunction is characterised by an onset of hypoactive sexual desire dysfunction following a period of time during which the person did not experience it and the desired response is currently absent or diminished in all circumstances, including masturbation. Situational, acquired hypoactive sexual desire dysfunction is characterised by an onset of hypoactive sexual desire dysfunction following a period of time during which the person did not experience it and the desired response is currently absent or diminished in some circumstances, with some partners, or in response to some stimuli, but not in other situations. In one embodiment, the hypoactive sexual desire dysfunction is unspecified hypoactive sexual desire dysfunction. In one embodiment, the hypoactive sexual desire dysfunction is lifelong, generalised hypoactive sexual desire dysfunction. In one embodiment, the hypoactive sexual desire dysfunction is lifelong, situational hypoactive sexual desire dysfunction. In one embodiment, the hypoactive sexual desire dysfunction is acquired, generalised hypoactive sexual desire dysfunction. In one embodiment, the hypoactive sexual desire dysfunction is acquired, situational hypoactive sexual desire dysfunction.

In one embodiment of the present disclosure, the hypoactive sexual desire dysfunction is characterised by:
(i) reduced or absent spontaneous desire,
(ii) reduced or absent responsive desire to erotic cues and stimulation, and/or
(iii) inability to sustain desire or interest in sexual activity once initiated.

In one embodiment of the present disclosure, the inability to sustain desire or interest in sexual activity has occurred episodically or persistently over a period at least several months, such as 3, 4, 5, 6, 7, or 8 months, and/or is associated with clinically significant distress.

Sexual arousal dysfunctions include difficulties with the physiological or the subjective aspects of sexual arousal. In one embodiment of the disclosure, the FSD is sexual arousal dysfunction (female sexual arousal dysfunction or FSAD). In one embodiment of the disclosure, the FSD is female sexual interest/arousal disorder/dysfunction (FSIAD). Female sexual arousal dysfunction (FSAD) is characterised by absence or marked reduction in response to sexual stimulation in women, as manifested by any of the following: 1) Absence or marked reduction in genital response, including vulvovaginal lubrication, engorgement of the genitalia, and sensitivity of the genitalia; 2) Absence or marked reduction in non-genital responses such as hardening of the nipples, flushing of the skin, increased heart rate, increased blood pressure, and increased respiration rate; 3) Absence or marked reduction in feelings of sexual arousal (sexual excitement and sexual pleasure) from any type of sexual stimulation. The absence or marked reduction in response to sexual stimulation occurs despite the desire for sexual activity and adequate sexual stimulation, has occurred episodically or persistently over a period at least several months, and is associated with clinically significant distress.

In one embodiment, the female sexual arousal dysfunction (FSAD) is lifelong female sexual arousal dysfunction or acquired female sexual arousal dysfunction. In one embodiment the female sexual arousal dysfunction is generalised female sexual arousal dysfunction or situational female sexual arousal dysfunction. In one embodiment of the disclosure, the female sexual arousal dysfunction is unspecified female sexual arousal dysfunction. Lifelong, generalised female sexual arousal dysfunction is characterised by the subject having always experienced female sexual arousal dysfunction from the time of initiation of relevant sexual activity and the desired response is currently absent or diminished in all circumstances, including masturbation. Lifelong, acquired female sexual arousal dysfunction is characterised by the subject having always experienced female sexual arousal dysfunction from the time of initiation of relevant sexual activity and the desired response is currently absent or diminished in some circumstances, with some partners, or in response to some stimuli, but not in other situations. Acquired, generalised female sexual arousal dysfunction is characterised by an onset of female sexual arousal dysfunction following a period of time during which the person did not experience it and the desired response is currently absent or diminished in all circumstances, including masturbation. Acquired, situational female sexual arousal dysfunction is characterised by an onset of female sexual arousal dysfunction following a period of time during which the person did not experience it and the desired response is currently absent or diminished in all circumstances, including masturbation. In one embodiment, the female sexual arousal dysfunctional is unspecified female sexual arousal dysfunction. In one embodiment, the female sexual arousal dysfunction is lifelong, generalised female sexual arousal dysfunction. In embodiment, the female sexual arousal dysfunction is lifelong, situational female sexual arousal dysfunction. In one embodiment, the female sexual arousal dysfunction is acquired, generalised female sexual arousal dysfunction. In one embodiment, the female sexual arousal dysfunction is acquired, situational female sexual arousal dysfunction.

In one embodiment, the female sexual arousal dysfunction (FSAD) is characterised by:
(i) absence or marked reduction in genital response, including vulvovaginal lubrication, engorgement of the genitalia, and sensitivity of the genitalia,
(ii) absence or marked reduction in non-genital responses such as hardening of the nipples, flushing of the skin, increased heart rate, increase blood pressure, and increased respiration rate, and/or
(iii) absence or marked reduction in feelings of sexual arousal, such as sexual excitement and sexual pleasure, from any type of sexual stimulation.

In one embodiment, the absence or marked reduction in response to sexual stimulation occurs despite the desire for sexual activity and adequate sexual stimulation, has occurred episodically or persistently over a period at least several months, such as 3, 4, 5, 6, 7, or 8 months, and/or is associated with clinically significant distress.

Orgasmic dysfunctions or female orgasmic disorder/dysfunction (FOD) refer to difficulties related to the subjective experience of orgasm. In one embodiment of the present disclosure, the FSD is orgasmic dysfunction. In one embodiment, the orgasmic dysfunction is not premature female orgasm.

In one embodiment, the orgasmic dysfunction is anorgasmia. Anorgasmia is characterised by the absence or marked infrequency of the orgasm experience or markedly diminished intensity of orgasmic sensations. In women, this includes a marked delay in orgasm, which in men would be diagnosed as Male Delayed Ejaculation. The pattern of absence, delay, or diminished frequency or intensity of orgasm occurs despite adequate sexual stimulation, including the desire for sexual activity and orgasm, has occurred episodically or persistently over a period at least several months, and is associated with clinically significant distress.

In one embodiment, the anorgasmia is lifelong anorgasmia or acquired anorgasmia. In one embodiment, the anorgasmia is generalised anorgasmia or situational anorgasmia. In one embodiment, the anorgasmia is unspecified anorgasmia. Lifelong, generalised anorgasmia is characterised by the subject having always experienced anorgasmia from the time of initiation of relevant sexual activity and the desired response is currently absent or diminished in all circumstances, including masturbation. Lifelong, situational anorgasmia is characterised by the subject having always experienced anorgasmia from the time of initiation of relevant sexual activity and the desired response is currently absent or diminished in some circumstances, with some partners, or in response to some stimuli, but not in other situations. Acquired, generalised anorgasmia is characterised by an onset of anorgasmia following a period of time during which the person did not experience it and the desired response is currently absent or diminished in all circumstances, including masturbation. Acquired, situational anorgasmia is characterised by an onset of anorgasmia following a period of time during which the person did not experience it and the desired response is currently absent or diminished in some circumstances, with some partners, or in response to some stimuli, but not in other situations. In one embodiment, the anorgasmia is lifelong, generalised anorgasmia. In one embodiment, the anorgasmia is lifelong, situational anorgasmia. In one embodiment, the anorgasmia is acquired, generalised anorgasmia. In one embodiment, the anorgasmia is acquired, situational anorgasmia.

In one embodiment, the anorgasmia is characterised by the absence or marked infrequency of the orgasm experience and/or markedly diminished intensity of orgasmic sensations.

In one embodiment, the absence or marked infrequency of the orgasm experience and/or markedly diminished intensity of orgasmic sensations occurs despite adequate sexual stimulation, including the desire for sexual activity and orgasm, has occurred episodically or persistently over a period at least several months, such as 3, 4, 5, 6, 7, or 8 months, and/or is associated with clinically significant distress.

In one embodiment, the FSD is sexual pain disorder. Sexual pain disorders refer to marked and persistent or recurrent difficulties related to the experience of pain during sexual activity in adult people, which are not entirely attributable to an underlying medical condition, insufficient lubrication in women, age-related changes, or changes associated with menopause in women and are associated with clinically significant distress.

In one embodiment, the sexual pain disorder is sexual pain-penetration disorder. Sexual pain-penetration disorder is characterised by at least one of the following: 1) marked and persistent or recurrent difficulties with penetration, including due to involuntary tightening or tautness of the pelvic floor muscles during attempted penetration; 2) marked and persistent or recurrent vulvovaginal or pelvic pain during penetration; 3) marked and persistent or recurrent fear or anxiety about vulvovaginal or pelvic pain in anticipation of, during, or as a result of penetration. The symptoms are recurrent during sexual interactions involving or potentially involving penetration, despite adequate sexual desire and stimulation, are not entirely attributable to a medical condition that adversely affects the pelvic area and results in genital and/or penetrative pain or to a mental disorder, are not entirely attributable to insufficient vaginal lubrication or post-menopausal/ age-related changes, and are associated with clinically significant distress.

In one embodiment the sexual pain-penetration disorder is lifelong sexual pain-penetration disorder or acquired sexual pain-penetration disorder. In one embodiment, the sexual pain-penetration disorder is generalised sexual pain-penetration disorder or situational sexual pain-penetration disorder. In one embodiment, the sexual pain-penetration disorder is unspecified sexual pain-penetration disorder. Lifelong, generalised sexual pain-penetration disorder is characterised by the subject having always experienced genito-pelvic pain or penetration disorder from the time of initiation of relevant sexual activity and the desired response is currently absent or diminished in all circumstances, including masturbation. Lifelong, situational sexual pain-penetration disorder is characterised by the subject having always experienced genito-pelvic pain or penetration disorder from the time of initiation of relevant sexual activity and the desired response is currently absent or diminished in some circumstances, with some partners, or in response to some stimuli, but not in other situations. Acquired, generalised sexual pain-penetration disorder is characterised by an onset of genito-pelvic pain or penetration disorder following a period of time during which the person did not experience it and the desired response is currently absent or diminished in all circumstances, including masturbation. Acquired, situational sexual pain-penetration disorder is characterised by an onset of genito-pelvic pain or penetration disorder following a period of time during which the person did not experience it and the desired response is currently absent or diminished in some circumstances, with some partners, or in response to some stimuli, but not in other situations. In one embodiment, the sexual pain-penetration disorder is lifelong, generalised sexual pain-penetration disorder. In one embodiment, the sexual pain-penetration disorder is lifelong, situational sexual pain-penetration disorder. In one embodiment, the sexual pain-penetration disorder is acquired, generalised sexual pain-penetration disorder. In one embodiment, the sexual pain-penetration disorder is acquired, situational sexual pain-penetration disorder.

In one embodiment, the sexual pain-penetration disorder is characterised by:
(i) marked or persistent or recurrent difficulties with penetration, including due to involuntary tightening or tautness of the pelvis floor muscles during attempted penetration,
(ii) marked and persistent or recurrent vulvovaginal or pelvic pain during penetration, and/or
(iii) marked and persistent or recurrent fear or anxiety about vulvovaginal or pelvic pain in anticipation of, during, or as a result of penetration.

In one embodiment, the sexual pain-penetration disorder has symptoms that:
(i) are recurrent during sexual interactions involving or potentially involving penetration, despite adequate sexual desire and stimulation,
(ii) are not entirely attributable to a medical condition that adversely affects the pelvic area and results in genital and/or penetrative pain or to a mental disorder,
(iii) are not entirely attributable to insufficient vaginal lubrication or post-menopausal or age-related changes, and
(iv) are associated with clinically significant distress.

Dyspareunia is a symptom of the genital system affecting females, caused by physical determinants. This symptom is characterised by recurrent genital pain or discomfort that occurs before, during, or after sexual intercourse, or superficial or deep vaginal penetration that is related to an identifiable physical cause, not including lack of lubrication. Confirmation is by medical assessment of physical causes. In one embodiment, the FSD is dyspareunia. In one embodiment, the FSD presents with dyspareunia.

In one embodiment, the sexual pain disorder is vulvodynia, or progressive vulvodynia. Vulvodynia is a persistent, unexplained pain in the vulva, which is the female genital area including the skin surrounding the opening of the vagina.

In one embodiment, the female sexual dysfunction is:
(i) associated with a medical condition, injury, or the effects of surgery or radiation treatment,
(ii) associated with psychological or behavioural factors, including mental disorders,
(iii) associated with use of psychoactive substance or medication,
(iv) associated with lack of knowledge or experience,
(v) associated with relationship factors,
(vi) associated with cultural factors, and/or
(vii) other specified aetiological considerations.

In one embodiment, the female sexual dysfunction is a hypoactive female sexual dysfunction.

In one embodiment, the female sexual dysfunction is not caused by another disease, such as a psychiatric disorder. In one embodiment, the female sexual dysfunction is not caused by injury to the genitals. In one embodiment of the disclosure, the subject is not under treatment with a medicament known to cause female sexual dysfunction.

In one embodiment of the present disclosure, the female sexual disorder is not a hyperactive female sexual disorder, such as premature female orgasm.

### Patient groups

In one embodiment of the present disclosure, the compound is capable of increasing the number of satisfying sexual events (SSE) in the subject. SSEs are used as an outcome measure in clinical trials of female sexual dysfunction, see Kingsberg et al. 2011. SSE may be assessed as outlined in Derogatis et al. 2021 or Clayton et al. 2016.

In one embodiment of the disclosure, the subject is female. In one embodiment, the subject is a mammal. In one embodiment of the disclosure, the subject is a human.

In one embodiment of the disclosure, the subject is post-menopausal.

In one embodiment of the present disclosure, the subject is over the age of 18, such as over the age of 20, such as over the age of 25, such as over the age of 30, such as over the age of 35, such as over the age of 40, such as over the age of 45, such as over the age of 50, such as over the age of 55, such as over the age of 60, such as over the age of 65, such as over the age of 70, such as over the age of 75.

In one embodiment, the subject is below the age of 40, such as below the age of 35, such as below the age of 30, such as below the age of 25, such as below the age of 20.

In one embodiment, the subject is 20 to 25 years of age, or 25 to 30, or 30 to 35, or 35 to 40, or 40 to 45, or 45 to 50, or 50 to 55, or 55 to 60, or 60 to 65, or 65 to 70, or 70 to 75, or older.

### Medical use

One embodiment of the present disclosure provides for a compound of formula (I), or a pharmaceutically acceptable salt thereof, for use in the treatment of female sexual dysfunction. One embodiment of the present disclosure provides for a composition comprising a compound of formula (I) or a pharmaceutically acceptable salt thereof, and at least one pharmaceutically acceptable carrier, excipient or diluent, for use in the treatment of female sexual dysfunction.

One embodiment of the present disclosure provides for a compound of formula (I), or a pharmaceutically acceptable salt thereof, for use in the treatment, prevention, or alleviation of female sexual dysfunction. One embodiment of the present disclosure provides for a composition comprising a compound of formula (I) or a pharmaceutically acceptable salt thereof, and at least one pharmaceutically acceptable carrier, excipient or diluent, for use in the treatment, prevention, or alleviation of female sexual dysfunction.

One embodiment of the present disclosure provides for use of a compound of formula (I) or a pharmaceutically acceptable salt thereof, for the manufacture of a medicament for the treatment of female sexual dysfunction. One embodiment of the present disclosure provides for use of a compound of formula (I) or a pharmaceutically acceptable salt thereof, for the manufacture of a medicament for the treatment, prevention, or alleviation of female sexual dysfunction.

One embodiment of the disclosure provides for a method of treatment, prevention, or alleviation of female sexual dysfunction in a subject in need thereof, said method comprising administering to the subject a therapeutically effective amount of a compound of formula (I) or a pharmaceutically acceptable salt thereof.

General and specific aspects of FSD may be evaluated using the Female Sexual Function Index (FSFI). The FSFI is a 19-item self-report inventory designed to assess female sexual function. It comprises six domains: desire (two items), arousal (four items), lubrication (four items), orgasm (three items), satisfaction (three items), pain (three items). In one embodiment, the subject prior to treatment had a female sexual function index (FSFI) of less than 26.55, such as 25.00 to 26.55, such as 20.00 to 25.00, such as 15.00 to 20.00, such as 10.00 to 15.00, such as 5.00 to 10.00, such as 2.00 to 5.00.

In one embodiment, the compound of the disclosure is capable of increasing the FSFI in the subject. In one embodiment, the compound is capable of increasing FSFI by at least 1 point, at least 2 points, at least 3 points, at least 4 points, at least 5 points, at least 6 points, at least 7 points, at least 8 points, at least 9 points, at least 10 points, at least 11 points, at least 12 points, at least 13 points, at least 14 points, at least 15 points, at least 16 points, at least 17 points, at least 18 points, at least 19 points, at least 20 points, at least 21 points, at least 22 points, at least 23 points, at least 24 points, at least 25 points, or at least 26 points.

In one embodiment, the compound of the disclosure is capable of increasing the FSFI to above 26.55.

The compound of the disclosure may also have an effect, such as a positive effect on any one or more of the six domains of the FSFI (desire, arousal, lubrication, orgasm, satisfaction and pain)), i.e. being capable of increasing the FSFI in any one or more of the six domains ( desire, arousal, lubrication, orgasm, satisfaction and pain) or any one question of each domain. In one embodiment, the compound of the present disclosure is able to increase the score of any one question of the FSFI questionnaire by 1, 2, 3, or 4 points.

The compound of the present disclosure may be especially efficient in improving the FSFI score of the pain domain. This is because the compound of the disclosure is a monoamine neurotransmitter reuptake inhibitor. The questionnaire contains 3 questions relating to pain, with a minimum score of 0 points and a maximum score of 6.0 points. In some embodiments, the questions are graded with a minimum score of 1 point and a maximum score of 5 points. In one embodiment, the compound of the present disclosure is capable of increasing the FSFI score of a subject in the pain domain. In one embodiment, the compound of the disclosure is capable of increasing the FSFI score of the pain domain by at least 0.3 points, at least 0.7 points, at least 1.0 points, at least 1.3 points, at least 1.7 points, at least 2.0 points, at least 2.3 points, at least 2.7 points, at least 3.0 points, at least 3.3 points, at least 3.7 points, at least 4.0 points, at least 4.3 points, at least 4.7 points, at least 5.0 points, at least 5.3 points, at least 5.7 points, or 6.0 points. In one embodiment, the compound of the disclosure is capable of increasing the score of FSFI question 17 (1^{st} pain question), such as increasing the score by 1, 2, 3, 4, or 5 points. In one embodiment, the compound of the disclosure is capable of increasing the score of FSFI question 18 (2^{nd} pain question), such as increasing the score by 1, 2, 3, 4, or 5 points. In one embodiment, the compound of the disclosure is capable of increasing the score of FSFI question 19 (3^{rd} pain question), such as increasing the score by 1, 2, 3, 4, or 5 points. The compound of the disclosure may also have an effect, such as a positive effect on any one or more of the other five domains (desire, arousal, lubrication, orgasm, and satisfaction), i.e. being capable of increasing the FSFI in any one or more of the other five domains (desire, arousal, lubrication, orgasm, and satisfaction).

The compound of the present disclosure may be especially efficient in improving the FSFI score of the desire domain (FSFI-D). The questionnaire contains 2 questions relating to sexual desire, each with a minimum score of 1 points and a maximum score of 5 points. In one embodiment, the compound of the present disclosure is capable of increasing the FSFI score of a subject in the desire domain. In one embodiment, the compound of the disclosure is capable of increasing the FSFI score of the desire domain by at least 0.3 points, at least 0.7 points, at least 1.0 points, at least 1.3 points, at least 1.7 points, at least 2.0 points, at least 2.3 points, at least 2.7 points, at least 3.0 points, at least 3.3 points, at least 3.7 points, at least 4.0 points, at least 4.3 points, at least 4.7 points, at least 5.0 points. In one embodiment, the compound of the disclosure is capable of increasing the score of FSFI question 1 (1^{st} desire question), such as increasing the score by 1, 2, 3, or 4points. In one embodiment, the compound of the disclosure is capable of increasing the score of FSFI question 2 (2^{nd} desire question), such as increasing the score by 1, 2, 3, or 4, points. The FSFI-D domain may be assessed by multiplying the score of each question in the desire domain by 0.6 and adding them together, forming a FSFI-D domain score range from 1.2 to 6.0. Thus, in one embodiment, the compound of the present disclosure is capable of increasing the score of the FSFI-D domain in a subject by 0.6 points, such as by 1.2, 1.8, 2.4, 3.0, 3.6, 4.2 or 4.8 points. In one embodiment, the compound is capable of increasing the score of the FSFI-D domain in a subject by 1.2, 2.4, 3.6 or 4.8 points.

In one embodiment, the compound of the present disclosure is capable of increasing the FSFI score of the arousal domain, or increasing the score of any one of the questions in the FSFI arousal domain, for example increasing the score of any one of questions 3 to 6 of the FSFI.

In one embodiment, the compound of the present disclosure is capable of increasing the FSFI score of the lubrication domain, or increasing the score of any one of the questions in the FSFI lubrication domain, for example increasing the score of any one of questions 7 to 10 of the FSFI.

In one embodiment, the compound of the present disclosure is capable of increasing the FSFI score of the orgasm domain, or increasing the score of any one of the questions in the FSFI orgasm domain, for example increasing the score of any one of questions 11 to 13 of the FSFI.

In one embodiment, the compound of the present disclosure is capable of increasing the FSFI score of the satisfaction domain, or increasing the score of any one of the questions in the FSFI satisfaction domain, for example increasing the score of any one of questions 14 to 16 of the FSFI.

General and specific aspects of FSD may be evaluated using the FSFI-6. The FSFI-6 is a 6-item Female Sexual Function Index questionnaire that measures sexual function in women (Isidori et al. 2010).

In one embodiment, the compound of the disclosure is capable of increasing the FSFI-6 in the subject. In one embodiment, the compound of the disclosure is capable of increasing the FSFI-6 by at least 1 point, at least 2 points, at least 3 points, at least 4 points, or at least 5 points.

One embodiment of the present disclosure provides for a compound of formula (I) or a pharmaceutically acceptable salt thereof, or a composition of the disclosure, for use in increasing FSFI in a subject suffering from female sexual dysfunction. One embodiment of the present disclosure provides for a compound of formula (I) or a pharmaceutically acceptable salt thereof, or a composition of the disclosure, for use in increasing FSFI-6 in a subject suffering from female sexual dysfunction.

One embodiment of the present disclosure provides for a use of a compound of formula (I) or a pharmaceutically acceptable salt thereof, or a composition of the disclosure, for the manufacture of a medicament for increasing the FSFI in a subject suffering from female sexual dysfunction. One embodiment of the present disclosure provides for a use of a compound of formula (I) or a pharmaceutically acceptable salt thereof, or a composition of the disclosure, for the manufacture of a medicament for increasing the FSFI-6 in a subject suffering from female sexual dysfunction.

One embodiment of the present disclosure provides for a method of increasing the FSFI in a subject suffering from female sexual dysfunction, said method comprising administering to said subject a compound of formula (I) or a pharmaceutically acceptable salt thereof, or a composition of the disclosure. One embodiment of the present disclosure provides for a method of increasing the FSFI-6 in a subject suffering from female sexual dysfunction, said method comprising administering to said subject a compound of formula (I) or a pharmaceutically acceptable salt thereof, or a composition of the disclosure.

General and specific aspects of FSD may be evaluated using the FSDS-DAO. The FSDS-DAO is a 15-item patient reported outcome (PRO) instrument/questionnaire that has be used in clinical trials to measure overall sexual function and grade the severity of female hypoactive sexual desire dysfunction (FHSDD) (Derogatis et al. 2008, Derogatis et al. 2021). In one embodiment, the subject prior to treatment had a female sexual function index (FSDS-DAO) of less than 60, such as 55 to 60, such as 50 to 55, such as 45 to 50, such as 40 to 45, such as 35 to 40, such as 30 to 35, such as 25 to 30, such as 20 to 25, such as 15 to 20, such as 10 to 15, such as 5 to 10, such as 0 to 10. In one embodiment, the subject prior to treatment had a female sexual function index FSDS-DAO of more than 10, such as more than 15, such as more than 20, such as more than 25, such as more than 30, such as more than 35, such as more than 40, such as more than 45, such as more than 50, such as more than 55.

In one embodiment, the compound of the disclosure is capable of decreasing the FSDS-DAO in the subject. In one embodiment, the compound is capable of decreasing FSDS-DAO by at least 1 point, at least 2 points, at least 3 points, at least 4 points, at least 5 points, at least 6 points, at least 7 points, at least 8 points, at least 9 points, at least 10 points, at least 11 points, at least 12 points, at least 13 points, at least 14 points, at least 15 points, at least 16 points, at least 17 points, at least 18 points, at least 19 points, at least 20 points, at least 21 points, at least 22 points, at least 23 points, at least 24 points, at least 25 points, at least 26 points, at least 27 points, at least 28 points, at least 29 points, at least 30 points, at least 31 points, at least 32 points, at least 33 points, at least 34 points, at least 35 points, at least 36 points, at least 37 points, at least 38 points, at least 39 points, at least 40 points, at least 41 points, at least 42 points, at least 43 points, at least 44 points, at least 45 points, at least 46 points, at least 47 points, at least 48 points, at least 49 points, at least 50 points, at least 51 points, at least 52 points, at least 53 points, at least 54 points, at least 55 points, at least 56 points, at least 57 points, at least 58 points, at least 59 points, or at least 60 points.

In one embodiment, the compound of the disclosure is capable of decreasing the question 13 score of the FSDS-DAO (FSDS-DAO Q13) in the subject. The compound of the present disclosure may be especially efficient in improving the question 13 of the FSDS-DAO (FSDS-DAO Q13) asks patients, "How often did you feel: Bothered by low sexual desire?", with a minimum score of 0 points scale of 0 (never) to 4 points (always). A decrease in the FSDS-DAO Q13 score over time denotes improvement in the level of distress associated with low sexual desire. In one embodiment, the compound of the disclosure is capable of decreasing the FSDS-DAO Q13 score by at least 0.3 points, at least 0.7 points, at least 1.0 points, at least 1.3 points, at least 1.7 points, at least 2.0 points, at least 2.3 points, at least 2.7 points, at least 3.0 points, at least 3.3 points, at least 3.7 points, or at least 4.0 points. In one embodiment, the compound of the disclosure is capable of decreasing the FSDS-DAO Q13 score by 1, 2, 3, or 4 points.

General and specific aspects of FSD may be evaluated using the Elemets of Desire Questionnaire (EDQ). The EDQ is a patient-reported outcome (PRO) developed to evaluate sexual desire. The EDQ addresses attributes of desire such as sexual desire, thoughts/fantasies about sex, and receptivity to sexual requests, with an objective of measuring the effectiveness of drugs for this condition with regard to more facets of the pathology. The EDQ is a 9-item PRO questionnaire intended to evaluate the efficacy of potential new treatments for women with FSD, HSDD or FSIAD. Items (questions) 1, 2, 3, 4, 6, and 8 in the EDQ is assessed on a 5 points scale with lower numbers indicating higher severity of dysfunction. Items 5 and 7 are assessed as a yes and no response. In one embodiment, the compound of the present disclosure is capable of increasing the EDQ score of a subject.

In one embodiment, the compound of the present disclosure is capable of increasing the score of any one question of the EDQ, such as question 1, 2, 3, 4, 5, 6, 7, 8 or 9. In one embodiment, one embodiment, the compound of the present disclosure is capable score of any one of questions 1, 2, 3, 4, 6, and 8 of the EDQ by 1, 2, 3, or 4 points.

Also disclosed is a compound of formula (I), or a pharmaceutically acceptable salt thereof, for use as described herein, wherein the compound is for increasing the genital temperature in the subject by administering a therapeutically effective amount of a compound of formula (I) or a pharmaceutically acceptable salt thereof.

In one embodiment of said disclosure, the increase in genital temperature is an increase in temperature to any one of: clitoris, vagina, labia, or any combination thereof. In one embodiment, the increase in genital temperature is an increase in clitoral temperature. In one embodiment, the increase in genital temperature is an increase in vaginal temperature. In one embodiment, the increase in genital temperature is an increase in vaginal and/or clitoral temperature

Also disclosed is a compound of formula (I), or a pharmaceutically acceptable salt thereof, for use as described herein, wherein the compound is for increasing genital blood flow in the subject by administering a therapeutically effective amount of a compound of formula (I) or a pharmaceutically acceptable salt thereof.

In one embodiment of said disclosure, the increase in genital blood flow is an increase of blood flow in any one of: clitoris, vagina, labia, or any combination thereof. In one embodiment, the increase in genital blood flow is an increase in clitoral blood flow.

In another aspect, the present disclosure provides a compound of formula (I), or a pharmaceutically acceptable salt thereof, for use as described herein, wherein the compound is for increasing sexual desire, sexual interest, sexual arousal, lubrication, sexual satisfaction, or a combination thereof in the subject by administering a therapeutically effective amount of a compound of formula (I), or a pharmaceutically acceptable salt thereof.

In another aspect, the present disclosure provides a compound of formula (I), or a pharmaceutically acceptable salt thereof, for use as described herein, wherein the compound is for reducing difficulty in reaching orgasm upon sexual stimulation or intercourse in the subject by administering a therapeutically effective amount of a compound of formula (I), or a pharmaceutically acceptable salt thereof.

One aspect of the present disclosure provides a compound of formula (I), or a pharmaceutically acceptable salt thereof, for use as described herein, wherein the compound is for reducing sexual pain in the subjectby administering a therapeutically effective amount of a compound of formula (I), or a pharmaceutically acceptable salt thereof.

### Examples

### Example 1: In vitro potency

### Materials and Methods

Compounds were tested for their ability to inhibit the reuptake of the monoamine neurotransmitters dopamine (DA), noradrenaline (NA), and serotonin (5-HT) in synaptosomes as described in WO 97/16451 (NeuroSearch A/S).

### Results

The test values are given as IC₅₀ (the concentration (µM) of the test substance which inhibits the specific binding of ³H-DA, ³H-NA, or ³H-5-HT by 50 %). See Table 1.

**Table 1: Test results obtained by testing exo-7-[(8-azabicyclo[3.2.1]octan-3-yl)oxy]-3-methoxy-chromen-2-one.**

| 5-HT-uptake IC₅₀ (µM) | DA-uptake IC₅₀ (µM) | NA-uptake IC₅₀ (µM) |
|---|---|---|
| 0.0029 | 0.07 | 0.0038 |

### Example 2: Evaluation of the effect of compound of formula (I) on rat female genital organs

### Materials and Methods

*Animals:* The experimental setup was run in two different sets of measurement procedures to avoid a variation given by each other. The first set of experiments was run in control female rats, and the second set was run in a depressed model called Flint Sensitive Line (FSL), which shares molecular similarities to major depressive patients.

*Anesthesia and general surgical procedure:* The rats were injected intraperitoneally with pentobarbital 50 mg/Kg. After 10 min in dark conditions a pinch of the interdigital area confirmed unresponsive of reflexes and the animal was prepared on a table, where legs were semi-extended by tape to fix the animal. The areas of interest (abdomen-inguinal and neck) were shaved and clean-disinfected with water and ethanol. After each study was completed, the animal was sacrificed by an overdose of pentobarbital, and the Atlanto-occipital joint was severed to guarantee a painless procedure for the animal.

*Measurements of clitoral blood flow and genital temperature:* A suture Nylon 4-0 with a needle was used to extend the urethra opening and expose the mucosa. To obtain the best visualization of the clitoris, we had a thread pulling at each side of the urethral wall, and then having another thread medial proximal to the urethra opening to extent cranially the skin and exposed in a better way the clitoral mucosal area (FIG. 1A). A calibrated laser Doppler flow probe (VP1T, Moor instruments-UK) was used to measure on the front-side of the clitoris, which was connected to the laser Doppler flow monitor; MOORVMS-LDF2 (Moor instruments-UK). A laser Doppler probe was positioned and fixed to a base and a crocodile clamp in order to avoid any movement. The tip of the calibrated probe was immersed in a water-based gel in the exposed area of the urethra-clitoris (FIG. 1B). Additionally, a right lateral dissection was performed to clean the jugular vein, which was used to insert another cannula used to perfuse different doses (0.3, 1, 3, 10 mg/Kg) of IP2015 or vehicle using a mechanical pump.

A calibrated laser Doppler flow probe (VP1T, Moor instruments-UK) was used to measure on the front-side of the clitoris, which was connected to the laser Doppler flow monitor; MOORVMS-LDF2 (Moor instruments-UK). Following a stabilization period of 10-20 min, baseline control measurements were obtained for 5 mins before infusion of vehicle or increasing doses (0.3, 1, 3, and 10 mg/Kg) of IP2015 via the jugular vein, and changes in blood flow were recorded. The infusion protocol was performed in sets of 1 min infusion + 4 min of further recording of blood flow changes recorded by the system. Electrical field stimulation of the cavernous nerve to the clitoris was utilized as a positive control as described below.

A thermographic FLIR camera (E96, InfraTec, Germany, Figure 1) was positioned at 50 cm distance from the area of interest (FIG. 1C) . Three areas (thorax, vagina, and urethral-clitoris) of interest were recorded by digital markers positioned using the Research Studio-FLIR, and data were extracted and imported to Excel. Following a stabilization period of 10-20 min, baseline control measurements were obtained for 5 mins before infusion of vehicle or increasing doses (0.3, 1, 3, and 10 mg/Kg) of IP2015 via the jugular vein, and changes in genital temperature were recorded.

*Measurements mean arterial pressure and heart rate:* The anaesthetized rats breathed spontaneously and body temperature was monitored continuously, and maintained at 37.5°^{C} by placing them on a heating blanket. A heparinized (100 IE mL⁻¹) polyethylene (PE) catheter (PE-50) connected to a pressure transducer (Disposable BP Transducer, AD Instruments, UK) was introduced into the carotid artery to measure MAP. Following a stabilization period of 10-20 min, continuous direct measurements of mean arterial pressure (MAP) and heart rate (HR) were obtained, registered, and analysed on a computerized data acquisition system (PowerLab, ADInstruments).

*Measurements of clitoral blood flow following electrical stimulation of the cavernous nerve to the clitoris:* Through a lower abdominal incision, the cavernous nerve to clitoris was isolated, and electrical stimulation was performed with a slender bipolar platinum electrode, which was connected to a S48 stimulator (Grass Instrument Co., Boston, MA, U.S.A.). To investigate whether stimulation of the nerve leads to hemodynamic changes in the clitorial tissue, the nerve was stimulated as previously described for male rats [12], with parameters eliciting the maximum amplitude of response (square wave pulses of 6 Volts, 10 Hz, 1 ms for 30 s), and flow was measured with laser Doppler. Clitoral blood flow was measured via laser Doppler as aforementioned.

### ResultsGenital blood flow

Laser Doppler measurements in the clitoral area of the female Sprague Daley rats, showed that infusion of IP2015 (0.3 to 10 mg/kg) increased the frequency, duration, and magnitude of blood flow in the clitoral area (FIG. 2). Further, average (FIG 6A) and peak (FIG 6B) clitoral blood flow were both significantly increased following infusion of IP2015 (0.3 to 10 mg/kg) compared to vehicle.

Peak clitoral blood flow following infusion of IP2015 (FIG 2A, 4.49V) was comparable to peak clitoral blood flow following electrical stimulation of the cavernous nerve to clitoris (FIG 5, 4.31 V)

### Genital temperature

Sexual arousal and desire has been demonstrated to increase genital temperature (Kukkonen *et al.,* 2007). The thermographic images at 4X magnification (FIG. 3A-C) and core temperature (FIG 3D) following infusion of IP2015 (0.3 to 10 mg/kg) and control. Extracted quantified data is presented in the histograms according to the different areas (thorax, vagina, and urethral-clitoris) of interest (FIG. 4A-C).

Measurements of the temperature using a thermographic camera revealed that basal surface temperatures were distinct depending on the region examined on the animal and changed during the infusion of increasing doses of IP2015 (FIG 3A-C, FIG. 4A-C). The recording conditions were optimized by zoom on the area of interest. Quantification at 4x zoom conditions, revealed infusion of increasing doses of IP2015 in depressed FSL rats caused dose-dependent increases in temperature in the vagina and clitoral area (FIG 3A-C, FIG. 4A-C). During the infusion of IP2015, the core temperature measured with a conventional anal thermometer was unchanged (FIG. 3D). The observed increases in genital temperature following IP2015 treatment, provides further evidence that IP2015 may induce an increase in sexual arousal and desire. Further, the observed increase in clitoral temperature following IP2015 treatment, provides further evidence that IP2015 may induce an increase in sexual arousal and desire specifically in FSD.

### Mean arterial pressure (MAP) and heart rate (HR)

Following infusion of IP2015 (0.3 to 10 mg/kg), MAP of the female Sprague Daley rats was comparable to vehicle (FIG 7A). Whereas, following infusion of IP2015 (0.3 to 10 mg/kg), HR of the female Sprague Daley rats was significantly increased to vehicle (FIG 7B).

### Conclusion

In summary, IP2015 dose-dependently increases blood flow and surface temperatures of genitalia in control Sprague Daley rats and a rat strain, FSL, with spontaneous depression. Further, the observed increase in genital blood flow and clitoral temperature following IP2015 treatment, provides further evidence that IP2015 may induce an increase in sexual arousal and desire. Further, the observed increase in clitoral temperature following IP2015 treatment, provides further evidence that IP2015 may induce an increase in sexual arousal and desire specifically in FSD. These findings support that IP2015 may improve sexual health in females.

### Example 3: Evaluation of the effect of compound of formula (I) on rat female sexual behaviour

### Materials and Methods

*Animals:* Female Sprague Dawley rats (12-15 weeks old) were housed in the animal facility in Universal Euro III type long width cages with standard wood bedding and space for 2 rats with a clean environment, and a comfortable temperature (22-23°C) with 12:12 h light-dark cycle starting at 11 am and they had free access to food and tap water.

*Synchronization of the sexual cycle in estrus of female rats:* Female Sprague Dawley rats with an average weight of 250 g were synchronized in the estrus phase of the sexual cycle by injecting with 250 µg prostaglandin F_{2α} (PGF_{2α}) subcutaneously at day 0 and day 3.

*Evaluating the effect of apomorphine vs IP2015 on sexual behavior:* At day 4, the rats were injected progesterone (1 mg/kg) 4 hours before any test was performed, and randomized to injection 2 before any testing was performed with vehicle (n=10), apomorphine (0.25 mg/kg) (n=10), and IP2015 (1 and 10 mg/kg, n=12 in each group). Apomorphine has previously been shown to increase, e.g., lordosis (Hamburger-Bar R *et al.,* 2011) and decrease, e.g., hops and darts (Snoeren *et al.,* 2011) in female rats, and was included as a positive control treatment. To investigate the sexual behavior, the synchronized female rats were paired with trained male rats and behaviour recorded by two cameras. The videos were quantified by a trained operator blinded for the treatments.

*Data analysis and statistics:* Statistical differences between vehicle, apomorphine and two different doses of IP2015 were analysed using one-way analysis of variance (ANOVA) followed by Tukey post-test. The assumptions of the models were investigated by inspecting Q-Q plots and data were logarithmically transformed when necessary in order to generate a Gaussian-distributed data set. In case where the data were skewed, the Brown-Forsythe test was applied. The ROUT method (Q=1%) was used to exclude data from the analysis. A P<0.05 was considered statistically significant. Post-tests were only done if F was significant and there was no variance inhomogeneity. All statistical analyses were performed using GraphPad Prism 9.5 software (GraphPad Software, CA, USA).

### Results

To investigate the effect of vehicle, IP2015, and apomorphine on the sexual behaviour of rats with estrus synchronized sexual cycles, we examined para-copulatory, e.g., hops, ear wiggling and darts, in female rats pre-treated with 250 µg PGF2α.

The term "estrus" refers to the phase of the estrous cycle in which a sexually mature, non-pregnant female is receptive to sexual advances from the male. Prostaglandin F2α (PGF2α ) is a naturally occurring luteolytic hormone that has also been utilized to synchronize estrus via shortening the length of the luteal phase.

Paracopulatory behavior, also called solicitation or proceptive behavior, is usually described as the species-specific behaviours displayed by an estrus female during sexual interaction in which she demonstrates sexual desire, arousal and interest and in turn initiates the establishment and maintenance of sexual interaction via encouraging the male to mate and regulates the pattern of copulation (Erskine, 1989).

Injection of vehicle did not change paracopulatory parameters (Figure 8). Injection of two different doses of IP2015 did not change ear wiggling (Figure 8A), but 10 mg/kg IP2015 increased the number of hops compared to apomorphine (Figure 8B). IP2015 dose-dependently increased darts compared with vehicle and also compared to apomorphine (Figure 8C). The observed increases in hops and darts in female rats treated with IP2015 demonstrates an IP2015-induced increase in desire, arousal and sexual interest. Injection of apomorphine did not change paracopulatory parameters (Figure 8).

### Conclusion

In summary, IP2015 dose-dependently increases sexual behaviors in female rats which is not dependent on the estrous cycle. The observed increases in hops and darts in female rats treated with IP2015 demonstrates an IP2015-induced increase in desire, arousal and sexual interest. These findings support that IP2015 may improve sexual health via increasing sexual behavior and desire in females.

### References

WO 97/30997.
WO 97/16451.
US 9,133,184 B2.
Allahdadi et al., Cardiovasc Hematol Agents Med Chem. 2009 Oct; 7(4): 260-269. Begrman, J.R., 2005. Physiology of female sexual function and dysfunction. International journal of impotence research, 17(1), pp.S44-S51.
Clayton et al., Bremelanotide for female sexual dysunctions in premenopausal women: a randomized, placebo-controlled dose-finding trial. Womens Health (Lond), 12:325-337.
Derogatis et al., J Patient Rep Outcomes, 2021, 5:100
Erskine, M.S., 1989. Solicitation behavior in the estrous female rat: a review. Hormones and behavior, 23(4), pp.473-502.
Hamburger-Bar R, Rigter H. Apomorphine: Facilitation of sexual behaviour in female rats. Eur J Pharmacol 1975;32. https://doi.org/10.1016/0014-2999(75)90304-0. Kingsberg SA, Althof SE. Satisfying sexual events as outcome measures in clinical trial of female sexual dysfunction. J Sex Med. 2011 Dec;8(12).
Isidori AM, Pozza C, Esposito K, Giugliano D, Morano S, Vignozzi L, Corona G, Lenzi A, Jannini EA. Development and validation of a 6-item version of the female sexual function index (FSFI) as a diagnostic tool for female sexual dysfunction. J Sex Med. 2010 Mar;7(3):1139-46.
Miller MK, Smith JR, Norman JJ, Clayton AH. Expert opinion on existing and developing drugs to treat female sexual dysfunction. Expert Opin Emerg Drugs. 2018;23(3):223-230.
Rosen R, Brown C, et al., 2000, The Female Sexual Function Index (FSFI): A Multidimensional Self-Report Instrument for the Assessment of Female Sexual Function, J Sex Marital Ther, 26(2):191-208.
Snoeren EMS, Chan JSW, De Jong TR, Waldinger MD, Olivier B, Oosting RS. A New Female Rat Animal Model for Hypoactive Sexual Desire Disorder; Behavioral and Pharmacological Evidence. J Sex Med 2011;8. https://doi.org/10.1111/j.1743-6109.2010.01998.x
T. M. Kukkonen, Y. M. Binik, R. Amsel, and S. Carrier, "Physiology: Thermography as a physiological measure of sexual arousal in both men and women," J. Sex. Med. 4, 93-105 (2007).

## Claims

1. A compound of formula (I), 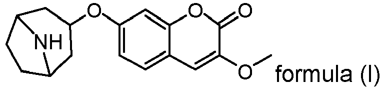
or a pharmaceutically acceptable salt thereof, for use in the treatment, prevention,
or alleviation of female sexual dysfunction in a subject.

2. The compound for use according to claim 1, wherein the compound is exo-7-[(8-azabicyclo[3.2.1]octan-3-yl)oxy]-3-methoxy-chromen-2-one hydrochloride.

3. The compound for use according to any one of the preceding claims, wherein the female sexual dysfunction is selected from the group consisting of:
(i) female sexual interest/arousal disorder,
(ii) hypoactive sexual desire dysfunction,
(iii) female sexual arousal dysfunction,
(iv) orgasmic dysfunction,
(v) sexual pain disorder, and
(vi) dyspareunia.

4. The compound for use according to any one of the preceding claims, wherein the female sexual dysfunction is female sexual interest/arousal disorder (FSIAD).

5. The compound for use according to any one of the preceding claims, wherein the female sexual dysfunction is hypoactive sexual desire dysfunction.

6. The compound for use according to any one of the preceding claims, wherein the female sexual dysfunction is orgasmic dysfunction, such as anorgasmia.

7. The compound for use according to any one of the preceding claims, wherein the female sexual dysfunction is sexual pain disorder.

8. The compound for use according to any one of the preceding claims, wherein the sexual pain disorder is vulvodynia, or progressive vulvodynia.

9. The compound for use according to any one of the preceding claims, wherein the subject prior to treatment had:
i) a female sexual function index (FSFI) of less than 26.55, such as 25.00 to 26.55, such as 20.00 to 25.00, such as 15.00 to 20.00, such as 10.00 to 15.00, such as 5.00 to 10.00, such as 2.00 to 5.00; and/or
ii) a Female Sexual Distress Scale-Desire/Arousal/Orgasm (FSDS-DAO) of less than 60, such as 55 to 60, such as 50 to 55, such as 45 to 50, such as 40 to 45, such as 35 to 40, such as 30 to 35, such as 25 to 30, such as 20 to 25, such as 15 to 20, such as 10 to 15, such as 5 to 10, such as 0 to 10.

10. The compound for use according to any one of the preceding claims, wherein the compound is capable of increasing the FSFI, such as increasing the FSFI score of any one of the domains desire, arousal, lubrication, orgasm, satisfaction and pain; increasing the FSFI-6 score, increasing the Elements of Desire Questionnaire (EDQ) score; and/or decreasing the Female Sexual Distress Scale-Desire/Arousal/Orgasm (FSDS-DAO) score in the subject.

11. The compound for use according to any one of the preceding claims, wherein the subject is a human female.

12. The compound for use according to any one of the preceding claims, wherein the subject is fertile, pre-menopausal, menopausal, or post-menopausal.

13. The compound for use according to any one of claims 1-11, wherein the subject is over the age of 18, such as over the age of 20, such as over the age of 25, such as over the age of 30, such as over the age of 35, such as over the age of 40, such as over the age of 45, such as over the age of 50, such as over the age of 55, such as over the age of 60, such as over the age of 65, such as over the age of 70, such as over the age of 75.

14. The compound for use according to claim 1, wherein the compound is for
i) increasing the genital temperature in the subject, such as an increase in temperature clitoris, vagina, labia, or a combination thereof; or
ii) increasing the genital blood flow in the subject, such as increase in the genital blood flow in the clitoris, vagina, labia, or a combination thereof.

15. The compound for use according to claim 1, wherein the compound is for
i) increasing sexual desire, sexual interest, sexual arousal, lubrication, sexual satisfaction, or a combination thereof;
ii) reducing difficulty in reaching orgasm upon sexual stimulation in a subject; or
iii) reducing sexual pain;
in the subject.

## Patentansprüche

1. Verbindung der Formel (I), 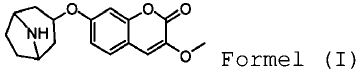 oder ein pharmazeutisch verträgliches Salz davon, zur Verwendung bei der Behandlung, Vorbeugung oder Linderung von weiblicher sexueller Dysfunktion in einem Subjekt.

2. Verbindung zur Verwendung nach Anspruch 1, wobei es sich bei der Verbindung um exo-7-[(8-Azabicyclo[3.2.1]octan-3-yl)oxy]-3-methoxy-chromen-2-on-Hydrochlorid handelt.

3. Verbindung zur Verwendung nach einem der vorhergehenden Ansprüche, wobei weibliche sexuelle Dysfunktion ausgewählt ist aus der Gruppe, bestehend aus:
(i) weiblicher Störung des sexuellen Interesses/der sexuellen Erregung,
(ii) hypoaktiver sexueller Luststörung,
(iii) weiblicher sexueller Erregungsstörung,
(iv) Orgasmusstörung,
(v) sexueller Schmerzstörung und
(vi) Dyspareunie.

4. Verbindung zur Verwendung nach einem der vorhergehenden Ansprüche, wobei es sich bei der weiblichen sexuellen Dysfunktion um eine weibliche Störung des sexuellen Interesses/der sexuellen Erregung (FSIAD) handelt.

5. Verbindung zur Verwendung nach einem der vorhergehenden Ansprüche, wobei es sich bei der weiblichen sexuellen Dysfunktion um eine hypoaktive sexuelle Luststörung handelt.

6. Verbindung zur Verwendung nach einem der vorhergehenden Ansprüche, wobei es sich bei der weiblichen sexuellen Dysfunktion um eine Orgasmusstörung, wie Anorgasmie, handelt.

7. Verbindung zur Verwendung nach einem der vorhergehenden Ansprüche, wobei es sich bei der weiblichen sexuellen Dysfunktion um eine sexuelle Schmerzstörung handelt.

8. Verbindung zur Verwendung nach einem der vorhergehenden Ansprüche, wobei es sich bei der sexuellen Schmerzstörung um Vulvodynie oder progressive Vulvodynie handelt.

9. Verbindung zur Verwendung nach einem der vorhergehenden Ansprüche, wobei das Subjekt vor der Behandlung Folgendes aufwies:
i) einen Index der weiblichen Sexualfunktion (FSFI) von weniger als 26,55, wie 25,00 bis 26,55, wie 20,00 bis 25,00, wie 15,00 bis 20,00, wie 10,00 bis 15,00, wie 5,00 bis 10,00, wie 2,00 bis 5,00; und/oder
ii) einen Wert auf der Female Sexual Distress Scale-Desire/Arousal/Orgasm (FSDS-DAO) von weniger als 60, wie 55 bis 60, wie 50 bis 55, wie 45 bis 50, wie 40 bis 45, wie 35 bis 40, wie 30 bis 35, wie 25 bis 30, wie 20 bis 25, wie 15 bis 20, wie 10 bis 15, wie 5 bis 10, wie 0 bis 10.

10. Verbindung zur Verwendung nach einem der vorhergehenden Ansprüche, wobei die Verbindung in der Lage ist, den FSFI zu erhöhen, wie Erhöhen des FSFI-Werts in einer beliebigen der Domänen Verlangen, Erregung, Lubrikation, Orgasmus, Befriedigung und Schmerz; Erhöhen des FSFI-6-Wertes, Erhöhen des Werts des Elements of Desire Questionnaire (EDQ); und/oder Verringern des Werts auf der Female Sexual Distress Scale-Desire/Arousal/Orgasm (FSDS-DAO) bei dem Subjekt.

11. Verbindung zur Verwendung nach einem der vorhergehenden Ansprüche, wobei das Subjekt eine Frau ist.

12. Verbindung zur Verwendung nach einem der vorhergehenden Ansprüche, wobei das Subjekt fertil, prämenopausal, menopausal oder postmenopausal ist.

13. Verbindung zur Verwendung nach einem der Ansprüche 1-11, wobei das Subjekt älter als 18 Jahre ist, wie älter als 20 Jahre, wie älter als 25 Jahre, wie älter als 30 Jahre, wie älter als 35 Jahre, wie älter als 40 Jahre, wie älter als 45 Jahre, wie älter als 50 Jahre, wie älter als 55 Jahre, wie älter als 60 Jahre, wie älter als 65 Jahre, wie älter als 70 Jahre, wie älter als 75 Jahre.

14. Verbindung zur Verwendung nach Anspruch 1, wobei die Verbindung zu Folgendem dient
i) Erhöhen der genitalen Temperatur des Subjekts, wie eine Erhöhung der Temperatur der Klitoris, der Vagina, der Schamlippen oder einer Kombination davon; oder
ii) Erhöhen der genitalen Blutzirkulation des Subjekts, wie Erhöhung der genitalen Blutzirkulation in der Klitoris, der Vagina, den Schamlippen oder einer Kombination davon.

15. Verbindung zur Verwendung nach Anspruch 1, wobei die Verbindung zu Folgendem dient
i) Steigern des sexuellen Verlangens, des sexuellen Interesses, der sexuellen Erregung, der Lubrikation, der sexuellen Befriedigung oder einer Kombination davon;
ii) Verringern der Schwierigkeit beim Erreichen eines Orgasmus nach sexueller Stimulation des Subjekts; oder
iii) Verringern von sexuellen Schmerzen;
bei dem Subjekt.

## Revendications

1. Composé de formule (I), 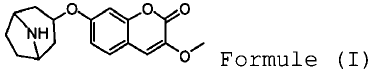 ou un sel pharmaceutiquement acceptable de celui-ci, pour une utilisation dans le traitement, la prévention ou le soulagement d'un dysfonctionnement sexuel féminin chez un sujet.

2. Composé pour une utilisation selon la revendication 1, dans lequel le composé est le chlorhydrate d'exo-7-[(8-azabicyclo[3.2.1]octan-3-yl)oxy]-3-méthoxy-chromén-2-one.

3. Composé pour une utilisation selon l'une quelconque des revendications précédentes, dans lequel le dysfonctionnement sexuel féminin est sélectionné dans le groupe constitué par :
(i) le trouble de l'intérêt/de l'excitation sexuelle féminine,
(ii) le dysfonctionnement hypoactif du désir sexuel,
(iii) le dysfonctionnement de l'excitation sexuelle féminine,
(iv) le dysfonctionnement orgasmique,
(v) le trouble de la douleur sexuelle, et
(vi) la dyspareunie.

4. Composé pour une utilisation selon l'une quelconque des revendications précédentes, dans lequel le dysfonctionnement sexuel féminin est le trouble de l'intérêt/de l'excitation sexuelle féminine (FSIAD).

5. Composé pour une utilisation selon l'une quelconque des revendications précédentes, dans lequel le dysfonctionnement sexuel féminin est le dysfonctionnement hypoactif du désir sexuel.

6. Composé pour une utilisation selon l'une quelconque des revendications précédentes, dans lequel le dysfonctionnement sexuel féminin est le dysfonctionnement orgasmique, tel que l'anorgasmie.

7. Composé pour une utilisation selon l'une quelconque des revendications précédentes, dans lequel le dysfonctionnement sexuel féminin est le trouble de la douleur sexuelle.

8. Composé pour une utilisation selon l'une quelconque des revendications précédentes, dans lequel le dysfonctionnement sexuel féminin est la vulvodynie, ou une vulvodynie progressive.

9. Composé pour une utilisation selon l'une quelconque des revendications précédentes, dans lequel le sujet, avant le traitement, présentait :
i) un indice de fonction sexuelle féminine (FSFI) inférieur à 26,55, par exemple de 25,00 à 26,55, par exemple de 20,00 à 25,00, par exemple de 15,00 à 20,00, par exemple de 10,00 à 15,00, par exemple de 5,00 à 10,00, par exemple de 2,00 à 5,00 ; et/ou
ii) un score à l'échelle de détresse sexuelle féminine - désir/excitation/orgasme (FSDS-DAO) inférieur à 60, par exemple de 55 à 60, par exemple de 50 à 55, par exemple de 45 à 50, par exemple de 40 à 45, par exemple de 35 à 40, par exemple de 30 à 35, par exemple de 25 à 30, par exemple de 20 à 25, par exemple de 15 à 20, par exemple de 10 à 15, par exemple de 5 à 10, par exemple de 0 à 10.

10. Composé pour une utilisation selon l'une quelconque des revendications précédentes, dans lequel le composé est capable d'augmenter le FSFI, par exemple en augmentant le score FSFI de l'un quelconque des domaines désir, excitation, lubrification, orgasme, satisfaction et douleur ; en augmentant le score FSFI-6, en augmentant le score du questionnaire Elements of Desire (EDQ) ; et/ou en diminuant le score à l'échelle de détresse sexuelle féminine - désir/excitation/orgasme (FSDS-DAO) chez le sujet.

11. Composé pour une utilisation selon l'une quelconque des revendications précédentes, dans lequel le sujet est une femme humaine.

12. Composé pour une utilisation selon l'une quelconque des revendications précédentes, dans lequel le sujet est fertile, pré-ménopausé, ménopausé ou post-ménopausé.

13. Composé pour une utilisation selon l'une quelconque des revendications 1 à 11, dans lequel le sujet est âgé de plus de 18 ans, par exemple de plus de 20 ans, par exemple de plus de 25 ans, par exemple de plus de 30 ans, par exemple de plus de 35 ans, par exemple de plus de 40 ans, par exemple de plus de 45 ans, par exemple de plus de 50 ans, par exemple de plus de 55 ans, par exemple de plus de 60 ans, par exemple de plus de 65 ans, par exemple de plus de 70 ans, par exemple de plus de 75 ans.

14. Composé pour une utilisation selon la revendication 1, dans lequel le composé est destiné à
i) augmenter la température génitale chez le sujet, par exemple une augmentation de la température du clitoris, du vagin, des lèvres ou une combinaison de ceux-ci ; ou
ii) augmenter le flux sanguin génital chez le sujet, par exemple une augmentation du flux sanguin génital dans le clitoris, le vagin, les lèvres, ou une combinaison de ceux-ci.

15. Composé pour une utilisation selon la revendication 1, dans lequel le composé est destiné à
i) augmenter le désir sexuel, l'intérêt sexuel, l'excitation sexuelle, la lubrification, la satisfaction sexuelle ou une combinaison de ceux-ci ;
ii) réduire la difficulté à atteindre l'orgasme lors d'une stimulation sexuelle chez un sujet ; ou
iii) réduire la douleur sexuelle ;
chez le sujet.
